# EUROPÄISCHE PATENTSCHRIFT

(11) **EP 1 080 740 B1**
(45) Veröffentlichungstag und Bekanntmachung des Hinweises auf die Patenterteilung: **27.07.2005**
(21) Anmeldenummer: 00117989.4
(22) Anmeldetag: 22.08.2000
(51) Int. Cl.: A61M 1/02, A61M 1/32

(54) **Einrichtung für die Zuführung eines Gasgemisches in einen Behälter**
Device to introduce a gas mixture in a container
Dispositif pour introduire un mélange de gaz dans un récipient

(30) Priorität: 04.09.1999 DE 19942382
(43) Veröffentlichungstag der Anmeldung: 07.03.2001
(73) Patentinhaber: Patentverwertung, Hänsler, J., Dr. GmbH, 76473 Iffezheim (DE)
(72) Erfinder: Viebahn-Hänsler, Renate, Dr., 76473 Iffezheim (DE)
(74) Vertreter: Flosdorff, Jürgen, Dr.

(56) Entgegenhaltungen:
- EP-A- 0 385 320
- DE-A- 19 526 112

## Beschreibung

Die Erfindung richtet sich auf eine Einrichtung für die Zuführung eines O₂/O₃-Gasgemisches in einen Behälter, insbesondere auch beutelartigen Behälter, für die Erhaltung der Funktion oder Refunktionalisierung von Blutkonserven bzw. Erythrozyten-Konzentraten sowie für die Eigenbluttherapie.

Die Refunktionalisierung bzw. Erhaltung der Funktion von Blutkonserven, genau die Erhaltung der Funktion bzw. Refunktionalisierung der Erythrozyten in Blutkonserven und Erythrozyten-Konzentraten, ist deshalb wichtig, weil Erythrozyten die Aufgabe haben, im Blutkreislauf den Sauerstoff durch das Gefäßsystem des Menschen zu transportieren und ihn am Ort des Verbrauchs zur Verfügung zu stellen, was über den Mechanismus der reversiblen Oxigenierung des Hämoglobins geschieht. Dabei greift als desoxigenierende Substanz das 2,3-Diphosphoglycerat (2,3-DPG) regulierend in die Oxigenierung des Hämoglobins ein. In menschlichen Erythrozyten kommt 2,3-DPG in etwa derselben molaren Menge vor, wie Hämoglobin und vermindert die Sauerstoffaffinität des Hämoglobins um den Faktor 26. Ein Mangel an 2,3-DBG bewirkt demnach eine erhöhte Sauerstoffaffinität und damit eine verminderte Sauerstoffabgabe am Ort des Sauerstoffbedarfs. Deshalb spielt bei der Übertragung von Blut aus Blutkonserven und Erythrozyten-Konzentraten der 2,3-DBG-Gehalt eine besondere Rolle und kann als Maß für die Funktionalität der Erythrozyten angesehen werden. Dies, weil während der Lagerung die Sauerstoffaffinität des Hämoglobins aufgrund sinkendem 2,3-DPG-Spiegels ansteigt, so dass beispielsweise nach 10-tägiger Lagerzeit einer Vollblutkonserve der 2,3-DPG-Gehalt nur noch etwa 10 Prozent des Ausgangswertes besitzt. Erythrozyten, die kein 2,3-DPG mehr besitzen, können nach einer Blutübertragung etwa erst nach 24 Stunden die Hälfte des Normalspiegels an 2,3-DPG erreichen und damit ihre soz. "alte" Funktion der Sauerstoffübertragung übernehmen. Beim Einsatz von Blutkonserven bestand also ein Problem darin, daß der 2,3-DPG-Gehalt durch den Alterungsprozeß oder Zugabe von Gerinnungshemmern und/oder Stabilisatoren absinkt und damit das Hämoglobin seine Fähigkeit der Sauerstoffübertragung verliert. Von diesem Sachverhalt ausgehend wurde gefunden, daß man durch Zuführung eines Ozon-Sauerstoff-Gemischs in das Blut einen gezielten Eingriff in den Erythrozyten-Stoffwechsel vornehmen kann, wodurch die Glykolyse wieder soweit aktiviert wird, daß erneut Diphosphoglycerat produziert wird und die Sauerstoffaffinität des Hämoglobins verringert wird, d.h. die Funktionalität der Erythrozyten wieder hergestellt bzw. aufrechterhalten wird.

Ferner ist es bekannt, im therapeutischen Dosierungsbereich bei der sog. "großen Eigenblutbehandlung" Ozon einzusetzen, obwohl das bei dieser Behandlung verwendete Frischblut in der Regel keinen Abfall der 2,3-DPG-Werte zeigt.

Es ist auch bekannt, daß bei der Ozonisierung von Blut durch Einleitung eines O₂/O₃-Gasgemisches die gleichmäßige Verteilung der Glasbläschen im Blut, aber nicht nur diese, sondern auch eine Feinverteilung eine erhebliche Rolle spielt, weil nur kleine Gasbläschen weitgehend gleicher Größe und gleichmäßig verteilt auch eine gleichmäßige Ozonisierung des Bluts garantieren, während bei größeren Gasblasen leicht eine lokal erhöhte O₃-Konzentration eintritt, die zu einer Teilhämolyse führen kann. Bei einer feinen Verteilung kleiner und unter sich weitgehend gleich großer Gasbläschen dagegen kann verhindert werden, daß durch das Ozon, das im Bruchteil einer Sekunde eine Reaktion eingeht und nicht physikalisch gelöst wird, lokal eine überschießende Reaktion stattfindet, die zur Zerstörung einzelner roter Blutkörperchen führen würde, während in der Nähe oder weiter weg sich im Blut befindende rote Blutkörperchen nicht mehr für eine Reaktion zur Verfügung stehen würden. Die Wegstrecke, die ein O₃-Molekül zurücklegt, ist also extrem kurz, sodaß es wichtig ist, daß die Reaktionsoberfläche des Gases möglichst groß und sehr gleichmäßig verteilt ist. Mit einer gleichmäßigen Feinverteilung der wirksamen Komponente O₃ läßt sich auch eine Senkung des Anteils und damit auch eine Senkung des O₂-Anteils zur Verhinderung der Bildung von Radikalen verbinden.

Eine Einrichtung für die Zuführung eines Gasgemisches in einen Behälter ist in EP-A-0 385 320 offenbart.

Die Industrie geht schon seit einiger Zeit mehr und mehr dazu über, Blut nicht mehr in Flaschen bereit zu halten, oder Flaschen für die Blutaufnahme vorzusehen, sodaß bisher angewandte Methoden für die Feinverteilung des O₃ z.B. mittels eines nahe des Flaschenbodens sich erstreckenden Maschensiebes, das das von oben einströmende Gas veranlassen soll, von der Unterseite des Siebes zurückzuströmen und sich dadurch mit Blut durchzumischen, nicht in Frage kommen. Abgesehen davon, daß ein solches Maschengitter sich nicht als geeignet herausstellte, eine gleichmäßige Feinverteilung des O₂/O₃-Gasgemisches zu garantieren, ist es bei den im Handel befindlichen Beuteln für die Aufnahme von Blut nicht möglich, ein solches Gitter anzubringen.

Hier setzt die Erfindung ein, mit der sich Ozon im Blut sehr fein in einem Behälter, insbesondere auch einem Behälter in Form eines Kunststoffbeutels, verteilen läßt, weil die erfindungsgemäße Einrichtung es gestattet, den beteiligten Abschnitt für die Zuführung eines O₂/O₃-Gasgemisches in den Behälter erst einzubringen, wenn der Beutel in Benutzung genommen wird.

Die Erfindung ist an einem, keinen Anspruch auf Vollständigkeit erhebenden Ausführungsbeispiel zur Anschauung gebracht und anhand dieses Beispiels nachfolgend beschrieben. Es stellen dar:
- Fig. 1: eine Einrichtung nach der Erfindung in vergrößertem Maßstab und im Nichtgebrauchszustand oder Zustand vor der Einführung in z.B. einen Blutbeutel,
- Fig. 2: die Einrichtung nach Fig. 1 im Maßstab der Fig. 1 im Gebrauchszustand,
- Fig. 3: das untere Ende einer Einrichtung nach den Fig. 1 und 2 in abgewandelter Ausführungsform und in einem gegenüber den Fig. 1 und 2 weiter vergrößertem Maßstab.

In der Zeichnung ist die erfindungsgemäße Einrichtung zur Vereinfachung der Darstellung nicht maßstabsgerecht wiedergegeben, sondern das Verhältnis Durchmesser : Länge in der Weise verzerrt, daß der Durchmesser im Verhältnis zur Länge vergrößert wurde.

Hauptteil der Einrichtung nach der Erfindung ist ein Gaszuführungsröhrchen 1, mit dem ein von oben (Pfeil A) einströmendes, von einem Ozongenerator erzeugtes O₂/O₃-Gasgemisch einem Blutbehälter zugeführt wird. Da die Erfindung insbesondere im Hinblick auf die Gas-Zuführung in einen Kunststoffbeutel konzipiert wurde, steht nachfolgend in dieser Beschreibung der Begriff "Beutel" für alle geeigneten Behälter.

Mit 2 ist allgemein ein zum Gaszuführungsröhrchen 1 gehörendes, gegebenenfalls auswechselbares Anschlußstück für die Verbindung eines O₂/O₃-Gas leitenden, vom Ozon-Generator kommenden Schlauches mit dem Röhrchen bezeichnet. An das Anschlußstück 2 schließt sich ein als Verteilungskammer 3 dienender Raum an, der auch kürzer als dargestellt sein kann.

In eine untere Abschlußplatte 4 des Röhrchens 1 ist ein Bündel von Hohlfasern 5 eingesetzt. In der Zeichnung sind aus Vereinfachungsgründen nur fünf Hohlfasern 5 eingezeichnet, aber tatsächlich sind mehr Hohlfasern 5 zu einem Bündel vereinigt, z.B. für ein Röhrchen 1 eines Innendurchmessers von etwa 2,5 bis 3,5 mm acht bis zehn Hohlfasern eines Innendurchmessers von etwa 0,2 mm bis etwa 0,3 mm. Damit strömt das O₂/O₃-Gasgemisch durch 2 ein, geht durch die Druckkammer 3 und von dieser in die Hohlfasern 5, durch deren Enden es aus- bzw. in das Blut einströmt (Pfeil B).

Wie dargestellt sind die Hohlfasern 5 vor dem Einsetzen in die Abschlußplatte 4 derart vorgespannt, daß sie bestrebt sind, sich insgesamt kelchförmig auseinander zu spreizen. Dabei können sie auch so angeordnet sein, daß ihre freien Enden in der Verteilungsstellung auf mehreren Kreisen, z.B. zwei Kreisen liegen.

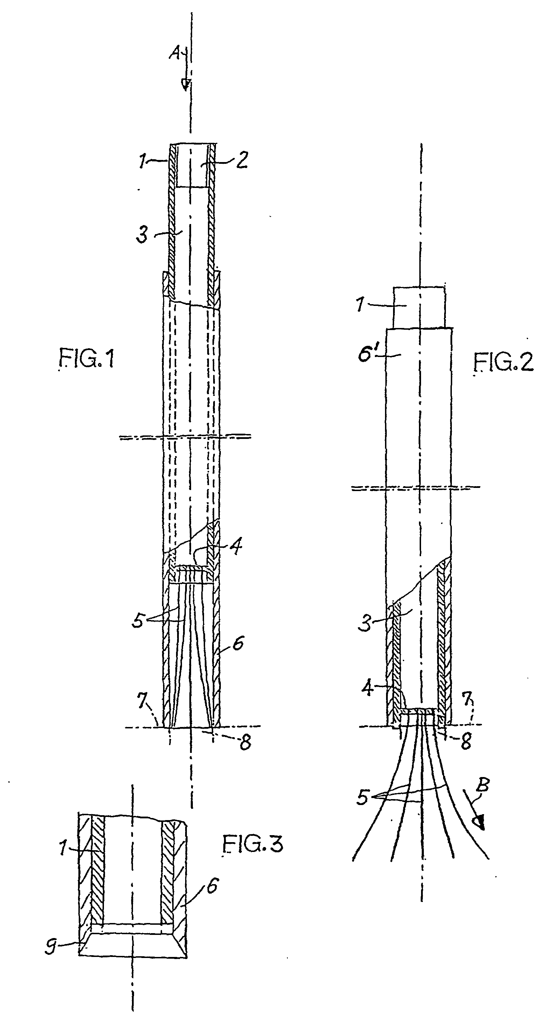

Falls der an der Zuführungsstelle verstärkte Beutel 7 eine oder mehrere Öffnungen 8 aufweist, wird die erfindungsgemäße Einrichtung mit der Schiebehülse 6 auf den Öffnungsrand aufgesetzt und auf das Gaszuführungsröhrchen 1 von oben in Pfeilrichtung A Druck ausgeübt, wodurch sich gemäß Fig. 2 das Röhrchen 1 durch die Schiebehülse 6 in den Behälter soweit durchdrücken läßt, bis die Hohlfasern 5 sich spreizen und ihre Enden im Beutel für die Feinverteilung an der richtigen Stelle, meist möglichst weit unten, liegen.

Für den Fall, daß keine oder keine passende Öffnung vorhanden ist, kann die Schiebehülse 6 an ihrem unteren beutelseitigen Ende mit einer Ringschneide 9 versehen sein, wie dies in Fig. 3 dargestellt ist, wobei die Hohlfasern 5 und die Abschlußplatte 4 weggelassen sind.

Der Raum 3 kann im Verhältnis zur Gesamtlänge des Röhrchens 1 bzw. der Hohlfasern 5 auch kürzer als dargestellt sein, sollte aber jedenfalls volumenmäßig so bemessen sein, daß eine sichere Verteilung des Gases auf alle Hohlfasern gewährleistet wird. Die Gesamteinrichtung nach der Erfindung sollte aus einem gegen das O₂/O₃-Gasgemisch inerten Kunststoff gefertigt sein, z.B. aus der Reihe der Fluor-Kunststoffe wie Polytetrafluorethylen (PTFE) oder der gesättigten Kohlenwasserstoffe wie Polypropylen (PP), Polyethylen (PE).

## Patentansprüche

1. Einrichtung für die Zuführung eines O₂/O₃-Gasgemisches in einen Behälter, insbesondere auch in einen aus Kunststoff bestehenden beutelartigen Behälter, für die Erhaltung der Funktion oder Refunktionalisierung von Blutkonserven bzw. Erythrozyten-Konzentraten sowie für die Eigenbluttherapie, wobei die Einrichtung ein Gaszuführungsröhrchen (1) enthält; in einem Bündel von für eine Spreizung vorgespannten Hohlfasern (5) endet, **dadurch gekennzeichnet, daß** über das Röhrchen (1) eine Schiebehülse (6) geschoben ist, die im Nichtgebrauchs- und Einführzustand das Röhrchenende überragt und die einzelnen Hohlfasern (5) an der vollen Spreizung hindert, während sie im hochgeschobenen Zustand (6') die Spreizung der Hohlfasern im Blut enthaltenden Beutel (7) zuläßt.

2. Einrichtung nach dem Anspruch 1,
**dadurch gekennzeichnet, daß** das für die Einführung in einen Blutbeutel bestimmte Ende der Schiebehülse (6) als Ringschneide (9) ausgebildet ist.

3. Einrichtung nach dem Anspruch 1,
**dadurch gekennzeichnet, daß** der Innendurchmesser des Röhrchens (1) etwa 2,5 mm bis 3,5 mm und der Innendurchmesser der Hohlfasern (5) etwa 0,2 mm bis 0,3 mm beträgt.

4. Einrichtung nach den Ansprüchen 1 bis 3,
**dadurch gekennzeichnet, daß** sie aus einem gegen Ozon inerten Kunststoff, z.B. PE, PP oder PTFE besteht.

## Claims

1. A device for introducing an O₂/O₃ gas mixture into a container, particularly also a bag-like container consisting of a plastic material, for maintaining the function or for refunctionalizing units of blood or erythrocyte concentrates and for autohemotherapy, the device containing a small gas-introducing tube (1) ending in a bundle of hollow fibers (5) that are pre-stressed for spreading, **characterized in that** a sliding sleeve (6) is slid over the small tube (1), said sliding sleeve projecting beyond the end of the small tube in the inoperative and introducing state and preventing the individual hollow fibers (5) from full spreading whereas in the pushed-up state (6') it allows spreading of the hollow fibers in the blood-containing bag (7).

2. The device according to claim 1,
**characterized in that** the end of the sliding sleeve (6) which is intended for introduction into a blood bag is configured as an annular cutting edge (9).

3. The device according to claim 1,
**characterized in that** the inner diameter of the small tube (1) is about 2.5 mm to 3.5 mm and the inner diameter of the hollow fibers (5) is about 0.2 mm to 0.3 mm.

4. The device according to claims 1 to 3,
**characterized in that** it consists of an ozone-inert plastic material, such as PE, PP or PTFE.

## Revendications

1. Dispositif pour introduire un mélange de gaz O₂/O₃ dans un récipient, en particulier, même dans un récipient en forme de poche en matière synthétique pour l'obtention de la fonction ou de la refonctionnalisation de stocks de sang ou respectivement de concentrés d'érythrocytes ainsi que pour la thérapie d'autotransfusion, où le dispositif contient un petit tube d'introduction de gaz (1) qui se termine en un faisceau de fibres creuses (5) pré-tendues pour un écartement **caractérisé en ce que** sur le petit tube (1) est poussée une douille coulissante (6) qui dépasse de l'extrémité du petit tube en position de non utilisation et d'introduction et empêche les fibres creuses individuelles (5) de s'écarter totalement, tandis qu'à l'état poussé (6') elle permet l'écartement des fibres creuses dans le sac contenant du sang.

2. Dispositif selon la revendication 1, **caractérisé en ce que** l'extrémité prévue pour une introduction dans un sac de sang de la douille coulissante (6) est configurée en cuvette.

3. dispositif selon la revendication 1, **caractérisé en ce que** le diamètre interne du petit tube (1) est d'environ 2,5 mm à 3,5 mm et le diamètre interne des fibres creuses (5) est d'environ 0,2 à 0,3 mm.

4. Dispositif selon les revendications 1 à 3, **caractérisé en ce qu'**il se compose d'un matériau inerte vis-à-vis de l'ozone, par exemple PE, PP ou PTFE.
